# EUROPEAN PATENT APPLICATION

(11) **EP 2 886 537 A1**
(43) Date of publication of application: **24.06.2015**
(21) Application number: 13306797.5
(22) Date of filing: 20.12.2013
(51) Int. Cl.: C07D 279/22, H01M 4/583, H01M 4/96, H01M 10/05, H01M 4/60, H01M 8/18, H01M 8/20, H01M 4/62, H01M 4/86, H01G 11/04, H01G 11/06, H01M 10/0525, H01M 10/054, H01M 10/0565, H01M 4/06

(54) **Modified carbon materials for use in energy storage devices**

(71) Applicant: Solvay SA, 1120 Bruxelles (BE); Institut Polytechnique de Grenoble, 38031 Grenoble Cedex (FR); Centre National de la Recherche Scientifique, 75794 Paris Cedex 16 (FR); INPG Entreprise SA, 38031 Grenoble (FR)
(72) Inventor: Godet-Bar, Thibault, 38000 Grenoble (FR); Druinot, Rémi, 38031 Grenoble (FR); Deronzier, Alain, 38240 Meylan (FR); Lepretre, Jean-Claude, 38340 Voreppe (FR); Sanchez, Jean-Yves, 38330 Saint-Ismier (FR)
(74) Representative: Vande Gucht, Anne

(57) **Abstract**

Electrodes for energy storage devices comprising modified carbon materials with organic redox moieties covalently attached to the carbon surface.

## Description

The present invention relates to the use of modified carbon materials for the manufacture of electrodes in energy storage devices and electrodes for energy storage devices comprising such modified carbon materials.

Electrodes for energy storage devices have to fulfil certain requirements in terms of electronic conductivity and in terms of electrode capacity for commercial applications.

One of the requirements is the capability to transport electrons from the redox center where the electrochemical reaction takes place through the electrodes to the current collectors.

Even when active materials with high electronic conductivity, such as graphite, are used as an electrode materials, it is common to use carbon as an additive to optimize the electronic conductivity of the electrode. The carbon in this application mainly ensures a better electronic percolation between the active material throughout the electrodes and up to the current collector, allowing the redox products to undergo along its volume the electron transfer. However, usual carbons do not exhibit redox properties thus contributing to a decrease of overall mass and volume capacity of the electrode material.

In the recent years, there have been intensive efforts in research and development of carbon materials with modified surfaces.

The modification of the carbon surface can be performed according to various routes. In accordance with a first strategy certain amount of surface atoms is turned into reactive groups, e.g. carboxyl groups, allowing further grafting of molecules onto the carbon surface. However, the reproducability of this approach is low and the oxidative nature of the process also is undesirable.

In accordance with a second strategy nitrene derivatives are used, which allow a thermal or photochemical process thereby grafting the targeted molecule to the surface.

Azomethine ylides similarly allow grafting molecules via an azirine moiety, but this grafting is reversible and thus not sufficiently stable

WO 92/13,983 discloses a process for modifying the surface of carbon-containing materials by electrochemical reduction of diazonium salts. The process is said to be particularly applicable to carbon fibers for composite materials. The carbon-containing material is used as a cathode in an electrolysis unit containing a diazonium salt solution in an aprotic solvent and by electrochemical reduction to a suitable potential, the aromatic group of the diazonium salt is bound to the carbon-containing material. Any aromatic group is considered to be suitable to be bound in this way to the carbon-containing material.

US 2005/34,629 discloses a process for preparing a carbon black product having an organic group attached to the carbon black. In one embodiment at least one diazonium salt reacts with a carbon black in the absence of an externally applied electric current sufficient to reduce the diazonium salt.

A similar process for the attachment of organic groups to the surface of a carbon material is disclosed in US patent 5,554,739.

WO 2004/41,915 discloses graphite/polymer hybrid composites with tailored structure obtained by a synthesis method based on stable free radical polymerization combined with polymerization compounding. The compounds as obtained in accordance with the claimed process are said to be suitable for use in bipolar plates for fuel cells and filter press electrolyzers as well as composite materials for the manufacture of automobiles and aircraft structures.

Lobo et al., Electroanalysis 1996, Vol. 8, issue 6, 591-596 discloses a comparative study of some phenoxazine and phenothiazine modified carbon-based electrodes for the ethanol determination. The organic material is spread on the surface of the carbon-based material.

Popescu et al., Revue Romaine de Chimie (2007) Vol. 52, Issues 8-9, pages 823-828, discloses a comparative study on the correlation between the electrochemical parameters of four phenothiazine derivatives. The derivatives are spread on the surface of the graphite electrode.

Muresan et al., Chem. Biochem. Eng. Quarterly, 2010, Vol. 24, issue 2, pages 159-166 discloses grafted electrodes modified with phenothiazine derivatives which are spread on the surface of the grafted electrode.

The electrodes obtained in accordance with the processes in the prior art referred to above are used for analytical purposes and not in energy storage devices and in most cases organic material used for modifying the carbon surface is simply spread over the carbon material, which leads to a physical adsorption of the material, but not to covalent bonding. Such materials are not suitable for use in electrodes for energy storage devices as it is undesirable that the materials attached to the carbon surface can be released from the surface quite easily in the process occurring in such an energy storage device leading to undesired reactions and processes in the cell.

Accordingly, it was an object of the present invention to provide electrodes for energy storage devices which show improved properties over the electrode systems of the prior art.

This object has been achieved with electrodes as claimed in claim 1.

Preferred embodiments of the present invention are set forth in the dependent claims and in the detailed specification hereinafter.

A further object of the present invention is the use of modified carbon materials comprising organic redox moieties covalently attached to the surface thereof for the manufacture of electrodes for energy storage devices, in particular rechargeable and non-rechargeable batteries and supercapacitors.

A third aspect of the present invention relates to certain azide compounds used for the modification of the carbon materials.

The first embodiment of the present invention is related to electrodes for energy storage devices comprising modified carbon materials with organic redox moieties covalently attached to the surface of the carbon material.

In the field of electrochemical energy storage, carbon plays an important role. The use of carbon materials is usually necessary to allow the electrons to be transported from the redox center, where the electrochemical reaction takes place, through the electrodes to the current collectors. Even when the active materials are highly electron conductive, carbon is normally used as an additive to optimize the electronic conductivity of the composite electrode. However, carbon used for that purpose would reduce the specific and volume capacity of the electrode and therefore it is desirable to either reduce the amount of carbon or to increase the specific and volume capacity of the carbon material.

The surface modification of carbon materials by covalent attachment of organic redox moieties allows both ensuring the electronic conductivity functions of the carbon materials and giving to the carbon extra functionalities, namely additional specific and volume capacity.

In accordance with a first preferred embodiment of the present invention, the redox moiety covalently attached bears an ionic function. Thereby materials are obtained showing a good electronic conductivity combined with good redox properties and ionic conductivities.

Alternatively, these functionalities can also be achieved in attaching separately redox and ion-pair moieties to the carbon surface.

In accordance with a first preferred embodiment, the redox moieties covalently attached to the carbon material are selected from molecules of formulae (1) or (2)

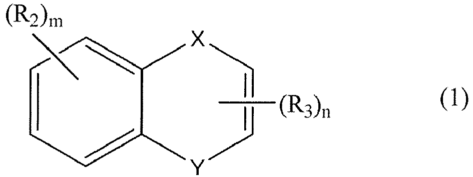

wherein X and Y, which may be the same or different, represent NR₁, O, S, SO or SO₂, and R₁ to R₃, which may be the same or different at each occurrence represent a C₁-C₃₀ hydrocarbyl group or a C₁-C₃₀ heterohydrocarbyl group, m may be 0 or an integer of from 1 to 3 and n may be 0, 1 or 2 or

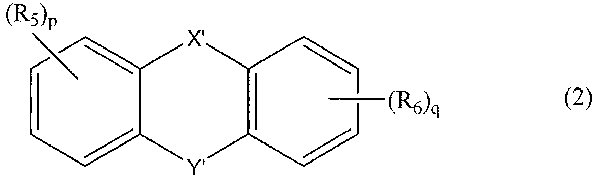

wherein X' and Y', which may be the same or different, represent NR₄, O, S, SO or SO₂, and R₄ to R₆, which may be the same or different at each occurrence, represent a C₁-C₃₀ hydrocarbyl group or a C₁-C₃₀ heterohydrocarbyl group and p and q, independent of each other may be 0 or an integer of from 1 to 3.

Preferred groups R₁ to R₆ are C₁-C₁₈ alkyl groups, C₁-C₁₈ alkoxy groups, C₆-C₃₀ aryl groups or C₃-C₃₀ heteroaryl groups. In accordance with a particularly preferred embodiment n and m respectively p and q are 0 and R₁ respectively R₄ is a C₁ to C₈ alkyl group, in particular methyl or ethyl, most preferred methyl.

The following compounds are given by way of example for suitable compounds which can be covalently attached to the surface of the carbon materials in accordance with the present invention.

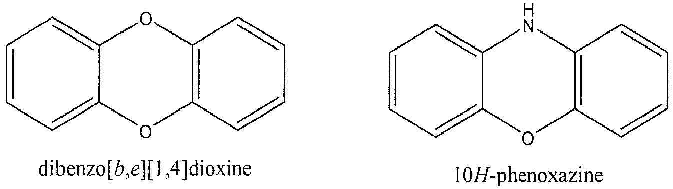

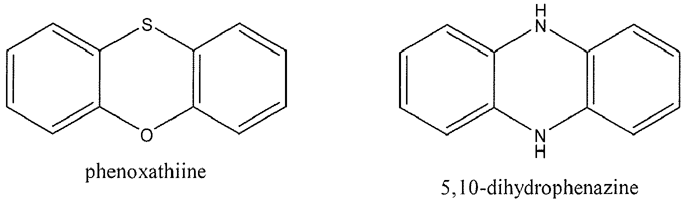

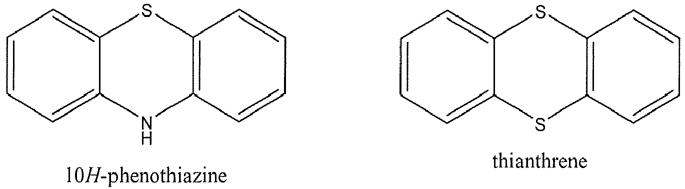

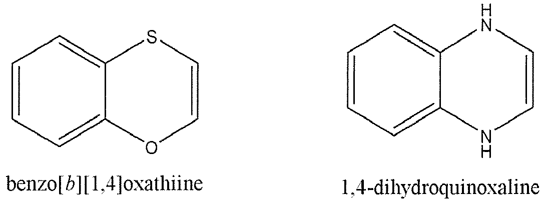

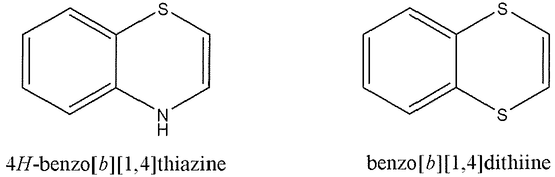

Preferred organic moieties which can covalently be attached to the surface of the carbon materials in accordance with the present invention are benzothiazines, benzoxazines, phenothiazines and phenoxazines.

Benzothiazines correspond to formula (1) with X being NR₁ and Y being S whereas benzoxazines correspond to formula I with X being NR₁ and Y being O.

Phenothiazines correspond to formula (2) with X' being NR₄ and Y' being S whereas phenoxazines correspond to formula (2) with X' being NR₄ and Y' being O.

Further preferred compounds which may be used in accordance with the present invention for the covalent attachment of redox moieties are N-Phenyl-thiazines or N-Phenyl-oxazines corresponding to the following formula with Y being S or O:

Another group of compounds which can be covalently attached to the 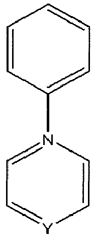 surface of the carbon materials are nitroxides or, as they should be more correctly named, aminoxyl radicals.

Aminoxyl radicals are derived from hydroxylamine and its derivatives by removal of the hydrogen atom from the hydroxy group and are in many cases stable radicals.

Some examples of this group of compounds are given below 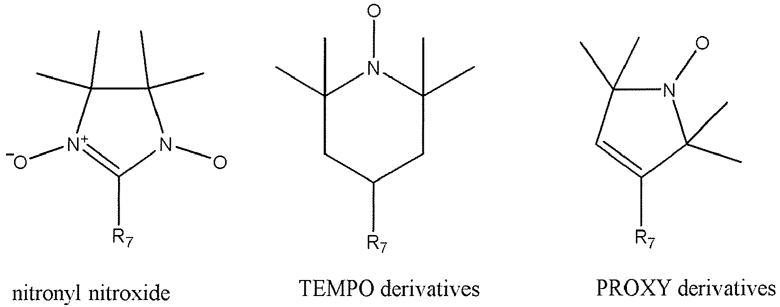 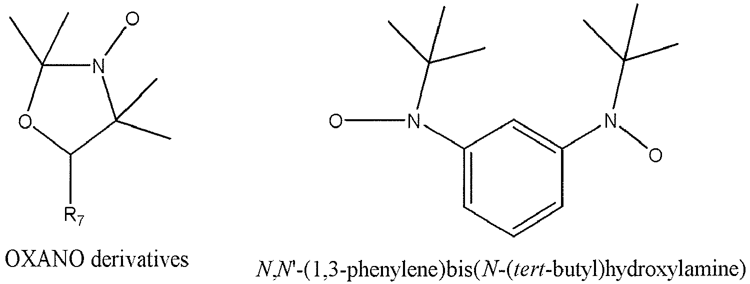 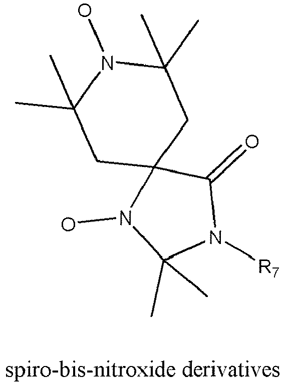 TEMPO in the above formulae stands for (2,2,6,6-tetramethylpiperidin-1-yl) oxidanyl, PROXY stands for 2,2,5,5-tetramethyl-1-(I1-oxidanyl)-2,5-dihydro-1H-pyrrole and OXANO stands for 2,2,5,5-tetramethyl-3-oxazolidinoxyl. Preferred substituents R₇ in the examples given above are aryl, in particular phenyl groups. It is apparent for the skilled person that the methyl groups shown in the examples can be replaced partly or fully by other alkyl groups, e.g. C₂-C₆ alkyl groups.

The compounds useful for attaching the redox moieties referred to above are known to the skilled person and in many cases commercially available. Thus, detailed information on their synthesis need not to be given here.

The covalent attachment of the redox moieties onto the surface of the carbon materials can be achieved by several processes, e.g. electrochemically, photochemically or by chemical reaction.

In many cases it has been found advantageous to attach the redox moieties by using diazonium or azide compounds.

A usually efficient method uses aryl diazonium salts through which a good control of the surface overlap can be achieved.

Diazonium salts and azides can be used to modify a great variety of different carbon materials like e.g. carbon black, graphite, glassy carbon.

In principle any carbon materials used in electrodes for energy storage devices can be grafted with diazonium salts or azide salts and thus the use of these salts for the grafting process to covalently attach the redox moieties to the carbon material is preferred. The skilled person will select the suitable carbon material depending on the specific application targeted based on his professional knowledge.

Processes to obtain the diazonium or azide salts of the compounds useful for attaching the redox moieties to the surface of the carbon materials are also known to the skilled person and have been described in the literature and thus no further details need to be given here.

The electrochemical grafting of the preferred diazonium salts can be achieved by cyclic voltammetry over a sufficient number of cycles with a potential lower than the reduction potential of the diazonium salt. Usually 5 cycles or more are used. The occurrence of the grafting can be seen in the voltammogram. Another possibility is to carry out the reduction by an electrochemical process applying a constant potential at the carbon electrode.

A suitable electrochemical process is described in Vautrin-UI et. al, Surface Science 600 (2006), 4801-4812. According to the process described in this reference, carbon electrodes after cleaning were dipped into a deaerated solution of a diazonium salt for various times in the range of from 10 min to 3 h, preferably for about one hour at room temperature. After the immersion in the solution, the substrates were rinsed with pure solvent (e.g. acetoonitrile) and thereafter with acetone to remove any organic material only weakly absorbed to the surface. This procedure is generally suitable for the electrochemical grafting.

Due to the reductive character of carbon materials, it is also possible to carry out the functionalization (covalently attaching the redox moieties through the diazonium or azide salts) by a chemical process.

The grafting rate of these processes can be enhanced by the addition of reductive agents, e.g. sodium borohydride or hypophosphorous acid in an aqueous or organic solvent.

Due to the reactivity of the radical versus the first deposited layer, the reduction of azide and diazonium salts, in particular of diazonium salts can be used to obtain multilayers of the grafted redox moieties on the surface of the carbon material.

The chemical functionalization of carbon by diazonium salts of e.g. phenothiazines (which represent a preferred group of compounds as disclosed hereinbefore) can be achieved by a method as described by Joselevich et al., Langmuir 24 (2008), 11711-11717 to which reference is made here for details. A detailed example is given later. The skilled person will adopt the process conditions depending on the starting materials based on his professional experience.

Azide salts can be grafted to carbon in accordance with Liu et al., J. Mat. Chem. 21 (2011), 3273-3276. A specific working example is given hereinafter. Again, the skilled person will adopt the process conditions depending on the staring materials based on his professional experience.

Another embodiment of the present invention is related to novel azide compounds of formula (3) or formula (4) 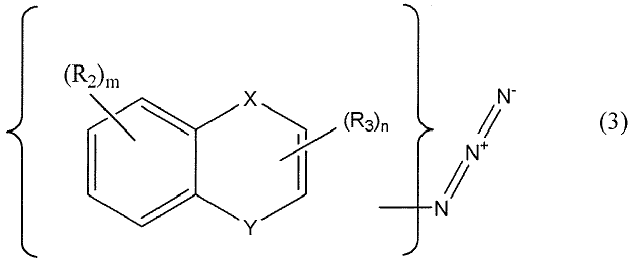 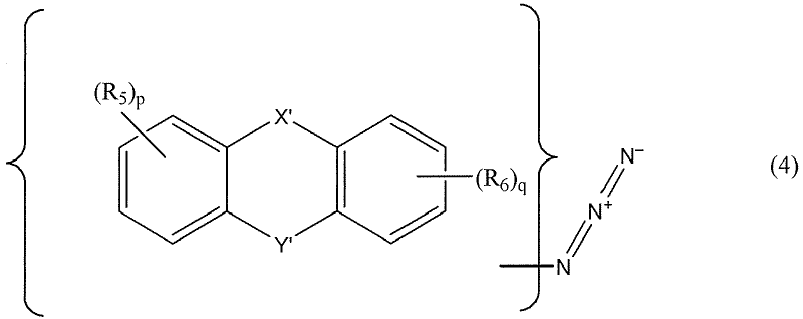 wherein the azide group may be attached to any of the carbon atoms in any of the aromatic rings and wherein X, X', Y and Y', which may be the same or different, represent NR₁, NR₄, O, S, SO or SO₂, and R₁ to R₆, which may be the same or different at each occurrence represent a C₁-C₃₀ hydrocarbyl group or a C₁-C₃₀ heterohydrocarbyl group and n, m, p and q, independent of each other may be 0 or an integer of from 1 to 3.

Preferred groups R₁ to R₆ are C₁-C₁₈ alkyl groups, C₁-C₁₈ alkoxy groups, C₆-C₃₀ aryl groups or C₃-C₃₀ heteroaryl groups. In accordance with a particularly preferred embodiment n and m respectively p and q are 0 and R₁ respectively R₄ is a C₁ to C₈ alkyl group, in particular methyl or ethyl, most preferred methyl.

Preferred azide compounds are compounds wherein X respectively X' is NR₁ respectively NR₄ and Y respectively Y' is S or O. R₁ and R₄ are preferably C₁-C₈ alkyl groups, particularly preferred methyl or ethyl groups, with methyl groups being most preferred.

The novel azide compounds in accordance with the present invention can be obtained by using processes principally known per se and described in more detail in the specification hereinafter and in the working examples.

The electrodes in accordance with the present invention, which comprise the modified carbon materials with organic redox moieties covalently attached to the surface thereof can be used in a grade variety of different energy storage devices like non--rechargeable batteries, rechargeable batteries, redox flow batteries or supercapacitors, e.g. hybrid supercapacitors.

Batteries based on alkali metals such as lithium, sodium or potassium or alkaline earth metals such as magnesium and calcium may be mentioned here. Such batteries can use as negative electrodes metals or intercalation materials e.g. lithiated graphite or alloys.

Just by way of example for rechargeable batteries lithium ion batteries, lithium polymer batteries, sodium ion batteries, magnesium ion batteries and calcium ion batteries may be mentioned here.

A lithium-ion battery (sometimes Li-ion battery or LIB) is a member of a family of rechargeable battery types in which lithium ions move from the anode to the cathode during discharge and back when charging. Li-ion batteries use an intercalated lithium compound as the electrode material, compared to the metallic lithium used either in non-rechargeable batteries or in lithium polymer batteries.

Lithium-ion batteries are common in consumer electronics. They are one of the most popular types of rechargeable battery for portable electronics, with one of the best energy densities, no or small memory effect, and only a slow loss of charge when not in use. Beyond consumer electronics, LIBs are also growing in popularity for military, electric vehicle and aerospace applications. For example, Lithium-ion batteries are becoming a common replacement for the lead acid batteries that have been used historically for golf carts and utility vehicles. Instead of heavy lead plates and acid electrolyte, the trend is to use a lightweight lithium/carbon anode and lithium iron phosphate cathode. Lithium-ion batteries can provide the same voltage as lead-acid batteries, so no modification to the vehicle's drive system is required.

Chemistry, performance, cost and safety characteristics vary across LIB types. Handheld electronics mostly use LIBs based on lithium cobalt oxide (LiCoO₂), which offers high energy density. Lithium iron phosphate (LFP), lithium manganese oxide (LMO), lithium nickel manganese oxide (LNMO) and lithium nickel manganese cobalt oxide (LNMC) offer lower energy density, but longer lives and increased safety. Such batteries are widely used for electric tools, medical equipment and other roles. Nickel manganese cobalt oxide (NMC) in particular is a leading contender for automotive applications. Lithium nickel cobalt aluminum oxide (NCA) and lithium titanate (LTO) are specialty designs aimed at particular applications.

The modified carbon materials with the covalently attached redox moieties can partly or fully replace the carbon black in the electrodes of those systems which is normally used to achieve the desired properties.

The redox modified carbon materials can also be used in organic redox polymer positive electrodes.

The redox modified carbon materials can also be used in positive electrodes consisting of blends of inorganic active materials such as LFP or NMC or in combination with organic redox polymers.

The amount of the modified carbon materials in the electrodes is not subject to particular limitations and usually is in the range of from 0.01 to 20, preferably from 0.02 to 10 and even more preferably from 0.03 to 8 wt% of the entire weight of the electrode.

Furthermore, the redox modified carbon materials can also be used as a component of the catholyte of redox-flow batteries.

A flow battery is a type of rechargeable battery where rechargeability is provided by two chemical components dissolved in liquids contained within the system and separated by a membrane. Ion exchange (providing flow of electrical current) occurs through the membrane while both liquids circulate in their own respective space. Cell voltage is chemically determined by the Nernst equation and ranges, in practical applications, from 1.0 to 2.2 Volts.

A flow battery is technically akin both to a fuel cell and an electrochemical accumulator cell (electrochemical reversibility). While it has technical advantages such as potentially separable liquid tanks and near unlimited longevity over most conventional rechargeables, current implementations are comparatively less powerful and require more sophisticated electronics for control.

A flow battery thus is a rechargeable fuel cell in which an electrolyte containing one or more dissolved electroactive elements flows through an electrochemical cell that reversibly converts chemical energy directly to electricity (electroactive elements are "elements in solution that can take part in an electrode reaction or that can be adsorbed on the electrode). Additional electrolyte is stored externally, generally in tanks, and is usually pumped through the cell (or cells) of the reactor, although gravity feed systems are also known. Flow batteries can be rapidly "recharged" by replacing the electrolyte liquid (in a similar way to refilling fuel tanks for internal combustion engines) while simultaneously recovering the spent material for re-energization.

In other words, a flow battery is just like an electrochemical cell, with the exception that the ionic solution (electrolyte) is not stored in the cell around the electrodes. Rather, the ionic solution is stored outside of the cell, and can be fed into the cell in order to generate electricity. The total amount of electricity that can be generated depends on the size of the storage tanks. One benefit to this design is that the cell can be recharged simply by changing out the tanks.

The redox modified carbon materials can also be used in supercapacitors and hybrid supercapacitors.

Supercapacitors (SC) comprise a family of electrochemical capacitors. Supercapacitors, sometimes also called ultracapacitors or electric double-layer capacitor (EDLC), don't have a conventional solid dielectric. The capacitance value of an electrochemical capacitor is determined by two storage principles, both of which contribute to the total capacitance of the capacitor.

Double layer-capacitance - electrostatic storage of the electrical energy achieved by separation of charge in a Helmholtz double layer at the interface between the surface of a conductive electrode and an electrolyte. The distance of the static separation of charge in a double-layer is of the order of a few Angströms (0.3-0.8 nm) which is extremely small and

Pseudocapacitance -Electrochemical storage of the electrical energy with electron transfer, achieved by redox reactions with specifically adsorbed ions from the electrolyte, intercalation of atoms in the layer lattice or electrosorption, underpotential deposition of hydrogen or metal atoms in surface lattice sites which result in a reversible faradaic charge-transfer.

Supercapacitors are divided into three families, based on the design of the electrodes:

Double-layer capacitors - with carbon electrodes or derivates with much higher static double-layer capacitance than the faradaic pseudocapacitance;

Pseudocapacitors - with electrodes out of metal oxides or conducting polymers with a high amount of faradaic pseudocapacitance; and

Hybrid supercapacitors - capacitors with special and asymmetric electrodes that exhibit both significant double-layer capacitance and pseudocapacitance, such as lithium-ion capacitors.

A hybrid supercapacitor is an electrochemical energy storage device that employs two different electrode types, the difference between the electrodes generally being in capacity or composition. Most commonly, one electrode is a redox (faradaic) electrode and the other a double-layer (non-faradaic) electrode.

Capacitors are widely used devices for storing electrical energy. Among the various types of capacitors are electrochemical capacitors and electrolytic capacitors.

Classical electrolytic capacitors consist of a series of combinations of two capacitors, separated by an electrolyte, and between which dielectric oxide film is formed adjacent to the surface of one or both of the electrodes.

An electrochemical capacitor is formed by electrochemical processes (double-layer charge storage or pseudo-faradaic charge storage) across an interface, such as the interface between an electrolyte and an electrode. Such capacitors rely on charge accumulation at an interface in order to store energy. Such capacitors do not rely on a dielectric oxide film for charge storage.

Compared to batteries as electrical energy storage system, capacitors have a lower energy density but a higher power density, i.e. the amount of energy per mass unit which can be stored is lower than with e.g. secondary batteries, but capacitors can be discharged and recharged very quickly thus providing a high power density which is required an advantageous for certain applications. Combinations of batteries and super capacitors provide a great discharge power which is useful in conjunction with devices for the starting of various engines.

The concept of hybrid super capacitors where one electrode is made from a rechargeable battery-type material and the other by activated carbon has been first introduced in aqueous electrolytes. Later, lithium intercalation compounds have been proposed for electrodes in symmetric capacitors using organic electrolytes.

The following examples show preferred embodiments of the present invention.

### Example 1

### Synthesis of MPDz (tetrafluoroborate of 10H-methylephenothiazine-3-diazonium)

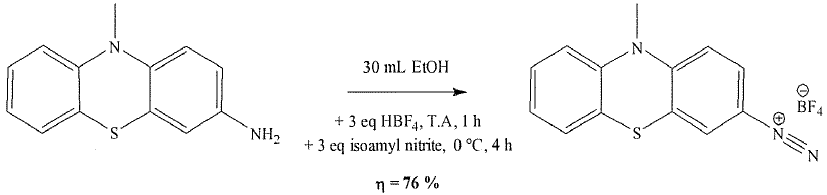

To a solution of Amino-MethylPhenothiazine, (AMP) (500 mg: 2.19 mmol) in 30 ml of absolute ethanol previously degassed for 30 min (argon), were added 3 eq-molar of tetrafluoroboric acid (900 µL, 6.57 mmol) dropwise. After 1 hour stirring in argon at room temperature, the solution was cooled at 0 °C before addition of 3 eq-molar of isoamyl nitrite (920 µL, 6.57 mmol). The mixture was kept under stirring in argon, at 0 °C for 4 h. 100 mL of diethyl ether (Et₂O) were added and the mixture maintained at 4°C for 24 h. The precipitate formed was washed with water, diethyl ether and ethyl acetate. The product was obtained in 76 % yield as a dark green powder. The structure was confirmed by NMR and elemental analysis.

Formula C₁₃H₁₀BF₄N₃S, molecular weight 327,11 g/mol

Elemental Analysis (theoretical) : %C = 47.73 ; %H = 3.08 ; %B = 3.31 ; %F = 23.23; %N = 12.85.

Elemental Analysis (experimental) %C = 45.37; %H = 3,19 ; %B = 2.96 ; %F = 20,49 ; %N = 10,55

**NMR ¹H 300 MHz in (CD₃)₂SO, δ in ppm :**
4;00 (Hₕ, s, 3H) ; 7.59 (H_{f}, dt, 1 H, ³J_{f-e,g} = 6.9 Hz, ⁴J_{f-d} = 1.0 Hz) ; 7.86-7,96 (H_{d,e}, m, 2H) ; 8,06 (Hₐ, d, 1 H, ³J_{a-b} = 9.4 Hz) ; 8.18 (Hg, dd, 1 H, ³J_{g-f} = 7.4 Hz, ⁴J_{g-e} = 0.5 Hz) ; 8.71 (H_{b}, dd, 1 H, ³J_{b-a} = 9.4 Hz, ⁴J_{b-c} = 2.2 Hz) ; 9.41 (H_{c}, d, 1 H, ⁴J_{c-b} = 2.2 Hz).

**NMR ¹³C 75 MHz in (CD₃)₂SO, δ in ppm** 37.05 (Cm, CH₃); 101.9 (C_{f}, C); 118.6 (Cₕ, CH); 119.2 (C_{b}, CH); 125.0 (Cₗ, C); 125.4 (C_{d}, CN₂⁺); 125.6 (Cⱼ, CH); 130.9 (C_{c}, CH); 134.1 (Cᵢ, CH); 134.8 (Cₑ, CH); 137.6 (Cₐ, C) ; 138.6 (Cₖ, CH) ; 145.9 (Cg, C).

**Infra red:**
**Vibrations** of aryl bonds: 819, 1,566 et 3,068 cm⁻¹
Vibrations of aryl bond C-N: 1,365 cm⁻¹
Vibrations of alkyl bond: 1,460 and 2,942 cm⁻¹
Vibration of C-N₂⁺bond: 2,234 cm⁻¹
Vibration of BF₄-bond: 1,027 cm⁻¹

### Example 2

### Synthesis of MPAz (10H-methylephenothiazine- sodium azide)

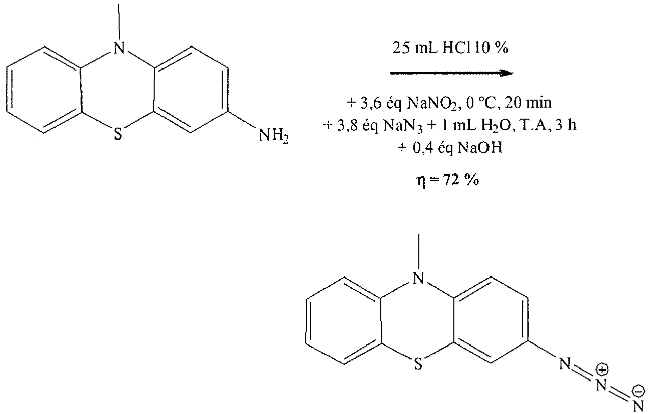

To a solution of AMP (50 mg; 0.219 mmol) in 25 ml of 10% HCl (6.2 mL HCl 37% + 18.8 mL distillated water) were added 3.6 eq-molar of sodium nitrite (55 mg, 0.790 mmol), at 0 °C for 20 min in argon. Sodium azide (3.8 eq-molar, 54 mg, 0.831 mmol) was added dropwise in 1 mL distilled water. After 2 hours stirring in argon at room temperature, NaOH was added (7 mg, 0.175 mmol) then 200 mL distillated water and 20 mL of Dichloromethane (DCM). The aqueous phase was extracted with 5x10 mL of DCM, the organic phases were combined, dried overNa₂SO₄, filtered and evaporated under vacuum.

The pure product was a red powder; yield 72%. The structure was confirmed by NMR and elemental analysis.

Formula C₁₃H₁₀N₄S, molecular mass 254,31 g/mol

**NMR ¹H 300 MHz in (CD₃)₂SO, δ in ppm:**
3.77 (Hₕ, s, 3H); 7.30 (H_{f}, dt, 1 H, ³J_{f-e,g} = 7.4 Hz, ⁴J_{f-d} = 0.9 Hz); 7.47 (H_{b}, dd, 1 H, ³J_{b-a} = 9.0 Hz, ⁴J_{b-c} = 2.7 Hz); 7.61 (H_{d}, dd, 1 H, ³J_{d-e} = 8.5 Hz, ⁴J_{d-f} = 1.0 Hz); 7.67 (Hₐ, d, 1 H, ³J_{a-b} = 9.0 Hz); 7.72 (He, ddd, 1 H, ³J_{e-f} = 7.2 Hz, ³J_{e-d} = 8.6 Hz, ⁴J_{e-g} = 1.6 Hz); 7.76 (H_{c}, d, 1 H, ⁴J_{c-b} = 2.7 Hz); 7.96 (Hg, dd, 1 H, ³J_{g-f} = 7.7 Hz, ⁴J_{g-e} = 1.5 Hz).

**NMR ¹³C 75 MHz in CDCl₃, δ in ppm:**
29.9 (Cₘ, CH₃); 115.7 (Cₕ, CH); 117.2 (C_{b}, CH); 120.7 (C_{c}, CH); 122,2 (Ce, CH); 123.7 (Cj, CH); 124.5 (Cₗ, C); 126.0 (C_{f}, C); 131.1 (Cᵢ, CH); 137.2 (Cₐ, C) ; 133.2 (Cₖ, CH) ; 134.1 (C_{d}, CN₃); 140.0 (C_{g}, C).

Infra red:
Vibrations of aryl bonds: 812, 1,588 and 3,063 cm⁻¹
Vibrations of aryl bond C-N: 1,351 cm⁻¹
Vibrations of alkyl bonds: 1,458 and 2,922 cm⁻¹
Vibration of C-N₃ bond: 2,102 cm⁻¹

### Example 3

### Electrochemical analysis

Acetonitrile (ACN) (from High Quality Rathburn HPLC (grade S, [H₂O]≤100ppm)) was used as received. The electrolyte, tetrabutylammonium perchlorate (TBAP) at 10⁻¹M in ACN was stored under argon atmosphere in dry glove box (Jaram).

### Device for electrochemistry

The device consisted of a potentiostat/galvanostat VSP Biologic science instruments, the software was EC-Lab. The analyses in ACN were performed, at ambient temperature, in a glove box Jaram under argon (O₂ < 5 ppm). A three-electrode system was used for the cyclic voltammetry. Reference electrode, Ag⁺/Ag, consisted of a silver wire immersed in a 10⁻² M solution of AgNO₃ in ACN + 10⁻¹ M of TBAP. Potential values measured were converted vs SHE (Standard Hydrogen Electrode) by adding 0.548 V or vs SCE (Saturated Calomel Electrode) by adding 0.298 V. Auxiliary Electrode consisted of a Pt wire immersed in ACN + 0.1 M TBAP and isolated from the measurement solution by a sintered glass. The working electrode (diam. 3 mm) used was a disk of glassy carbon of 0.283 cm² surface area. It was previously polished with a diamond preparation of 2 µm (Escil). Experiments are carried out in electrochemical cells Metrohm EA of 10 mL.

### Diazonium analysis

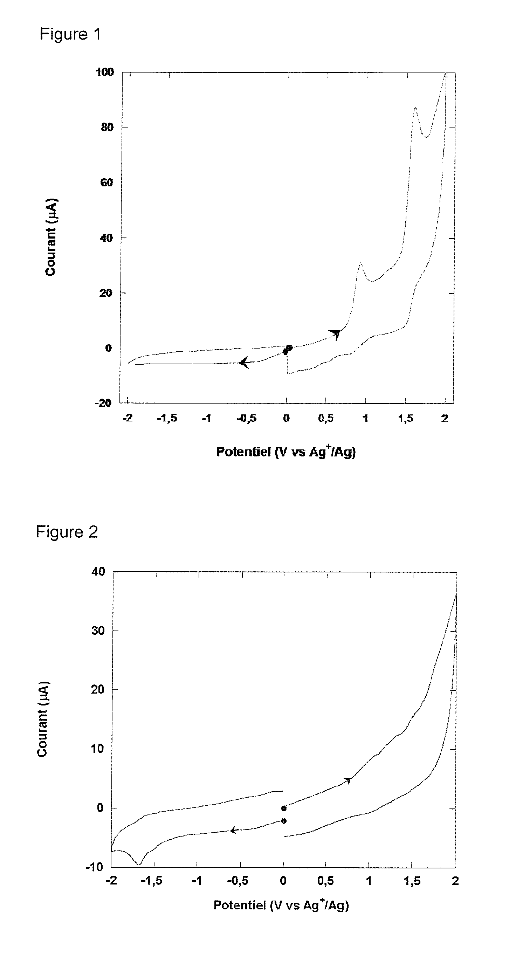

Figure 1 shows the cyclic voltammogram in oxidation and reduction of a 2.18 x 10⁻³M solution of MPDz on a glassy carbon electrode (diameter 3 mm) in ACN + TBAP 10⁻¹M.

The cyclic voltammetry CV showed, as expected, in the cathodic region an irreversible and broad reduction peak at Epc = -0.83V Ag/Ag⁺. This potential value is rather more negative compared to that measured for diazonium salts from literature (i.e. 0.75V for tolyl diazonium as example). The broadness of the cathodic peak is presumably due to some adsorption phenomena coupled to the electron transfer leading to a cathodic shift of the E_{pc} value. In the anodic region, the two successive oxidation steps centred on phenothiazine are observed at 0.866 and 1.537 V vs Ag⁺/Ag. Compared to pristine phenothiazine, one can observe a shift of about 450 mV towards the most anodic value of the two redox processes due to the presence of the electron withdrawing N₂⁺ moieties.

### Azide analysis

Fig. 2 shows the the cyclic voltammogram in oxidation and reduction of a 1.55 x 10⁻³M solution of MPAz on a glassy carbon electrode (diameter 3 mm) in ACN + TBAP 10⁻¹M

The cyclic voltammetry, despite the high purity of the starting product, did not exhibit the two anodic peaks corresponding to the two successive one-electron oxidation processes centered on phenothiazine. This particular behavior is due to some intense adsorption phenomenon coupled to electron transfer avoiding a clear detection of the redox signature. This attribution is sustained by the fact along cycling that one can observe the progressive increase of both anodic and cathodic currents due to some accumulation of the product at the surface of the electrode. On the other hand, upon washing the electrode with acetonitrile, the deposited material is easily removed showing that the accumulation does not involve a covalent grafting of the product. Azide analysis in oxidation does not evidence an electrochemical system. On the other hand, in reduction, a redox system is characterized at -1.630 V vs Ag⁺/Ag with a ΔEₚ of 94 mV. As the reverse signal in oxidation is very weak, the system is assumed to be irreversible.

### Example 4

### Electrochemical grafting on a disk electrode of glassy carbon (GC)

### Diazonium grafting

MPDz was grafted on GC electrode by performing a CV over 10 cycles between -2 et 0,8 V vs Ag⁺/Ag at 100 mV.s⁻¹.

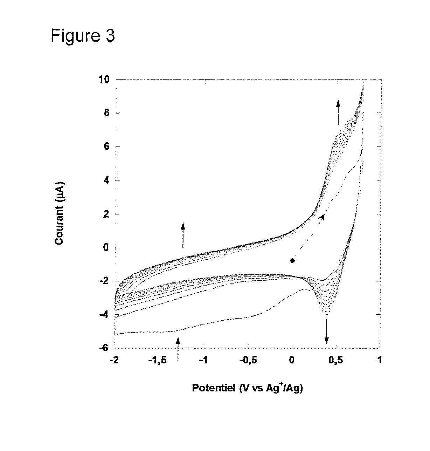

Fig. 3 shows the electrochemical grafting of MPDz on a glassy carbon electrode (diameter 3 mm) by CV (10 cycles) at 100 mV/s with a 2.18 x 10-³M solution of MPDz in ACN + TBAP 10⁻¹M.

Along cycling, a great increase of the characteristic signature of phenothiazine was observed at close potential to that of 10H-methylphenothiazine (MP) i.e. 0.424 V vs Ag⁺/Ag). After ten cycles the grafted electrode was transferred in an acetonitrile solution free of diazonium salt. The resulting CV shows unambiguously the presence of phenothiazine grafted onto the surface of the electrode as judged by a reversible system at 0.455V vs Ag⁺/Ag. Along cycling the signal was maintained whereas performing at low scan rate (i.e. 5mV.s⁻¹) the CV shared the same shape, confirming the phenothiazine to be covalently grafted on the vitreous carbon surface. The same behaviour was observed using carbon foam allowing to graft higher amounts of phenothiazine.

### Example 5 - Grafting by chemical route

### Diazonium salt

30 mg carbon Super C-65A were added to freshly distilled tetrahydrofurane (10mL) containing MPDz (10 mg; 3,06.10⁻⁵ mol). The mixture was maintained at room temperature during 24h.

After sonication the solid was washed using dichloromethane and CH₃CN in order to remove soluble phenothiazine derivatives to lead to 30 mg of final product. The presence of grafted phenothiazine was confirmed by infrared spectroscopy (758, 1,571 and 1,456 cm⁻¹) whereas functionalization rate of carbon by phenothiazine was determined by elemental analysis (i.e. %C = 97.66 ; %H = 0.61 ; %S = 0.20).

For the latter, it was estimated that close to 0.1 % of the carbon atom of the carbon particle bear a phenothiazine. Taking into account particle sizes are around 50nm, this means that roughly 25% of the surface area of carbon had been functionalized by the phenothiazine moieties.

### Azide grafting

To a 10 mL solution of MPAz (20 mg ; 7.86.10⁻⁵ mol) in o-dichlorobenzène 100 mg of carbon Super C-65 are added. The mixture is maintained at 90 °C during 72 h. After cooling, the black mixture is sonicated then the solid is washed with dichloromethane and acetonitrile to remove the unreacted MPAz.

The presence of grafted phenothiazine is confirmed by infrared spectroscopy (745, 1,591 and 1,460 cm⁻¹) whereas functionalization rate of carbon by phenothiazine is determined by elemental analysis (i.e. %C = 96.8 ; %H = 0.53 ; %S = 0.29.).

Thus, it has been estimated that close to 0.12% of the carbon atoms of the carbon particles bear a phenothiazine. This amount shows that carbon functionalization using azide derivative is more efficient compared to diazonium one, since more than one third of the surface area of carbon has been functionalized by the phenothiazine moieties.

### Example 6 - Electrochemical analysis

After analysis of the functionalization rate of carbons grafted by MP, the modified carbons were analyzed by cavity microelectrode, a method allowing the electrochemical signature of an insoluble compound to be recorded, without requiring its formulation in a ink to be deposited on a planar electrode.

### Diazonium grafted on carbon

The methyl phenothiazine grafted carbon was analyzed at 100 mV/s. The redox system was centered at 0.538 V vs Ag⁺/Ag with a ΔEₚ of 229 mV. The apparent redox potential of the electro-active species is shifted by +100 mV with regard to that created by electrochemical grafting. However, on the reverse scan, the cathodic peak potential, the E_{p,c} 0,423 V *vs* Ag⁺/Ag was close to that of the diazonium grafted on electrode after transfer i.e. 0.411 V *vs* Ag⁺/Ag.

### Azide grafted on carbon

The MP grafted on carbon via MPAz intermediary is analyzed at several sweep rates: 100, 50, 20 and 10 mV.s⁻¹. The apparent redox potentials and the peak potential differences for different sweep rates are gathered in the following table 1, showing that at low scan rate the redox system appears to be quite reversible:

**Table 1**

| Sweep rate /mV.s⁻¹ | E^{0'}/V vs Ag⁺/Ag | ΔEₚ /mV |
|---|---|---|
| 100 | 0.525 | 152 |
| 50 | 0.534 | 122 |
| 20 | 0.562 | 104 |
| 10 | 0.577 | 95 |

## Claims

1. Electrodes for energy storage devices comprising modified carbon materials with organic redox moieties covalently attached to the surface of the carbon material.

2. Electrodes in accordance with claim 1 wherein the redox moiety compounds are represented by the general formula (1) 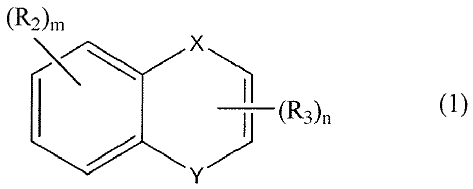
wherein X and Y, which may be the same or different, represent NR₁, O, S, SO or SO₂, and R₁ to R₃, which may be the same or different at each occurrence represent a C₁-C₃₀ hydrocarbyl group or a C₁-C₃₀ heterohydrocarbyl group, m may be 0 or an integer of from 1 to 3 and n may be 0, 1 or 2.

3. Electrodes in accordance with claim 1 wherein the redox moiety compounds are represented by the general formula (2) 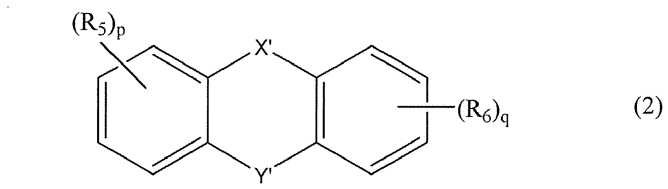
wherein X' and Y', which may be the same or different, represent NR₄, O, S, SO or SO₂, and R₄ to R₆, which may be the same or different at each occurrence, represent a C₁-C₃₀ hydrocarbyl group or a C₁-C₃₀ heterohydrocarbyl group and p and q, independent of each other may be 0 or an integer of from 1 to 3.

4. Electrodes for energy storage devices in accordance with claim 1 wherein the redox moiety bears an ionic function.

5. Electrodes for energy storage devices in accordance with claim 1 wherein ionic groups are bound to the surface of the carbon material in addition to the redox moieties.

6. Electrodes in accordance with claim 1 wherein the organic redox moieties are derived from benzothiazines, benzoxazines, phenothiazines, phenoxazines, N-phenyl thiazines or nitroxides.

7. Electrodes in accordance with claim 2 wherein the benzothiazines and benzoxazines are represented by the general formula (1) wherein X represents NR₁ and Y represents O or S and R₁ to R₃, which may be the same or different at each occurrence represent a C₁-C₃₀ hydrocarbyl group or a C₁-C₃₀ heterohydrocarbyl group, m may be 0 or an integer of from 1 to 3 and n may be 0, 1 or 2.

8. Electrodes in accordance with claim 3 wherein the phenothiazines and phenoxazines are represented by the general formula (2) wherein X' represents NR₄ and Y' represents O or S and R₄ to R₆, which may be the same or different at each occurrence represent a C₁-C₃₀ hydrocarbyl group or a C₁-C₃₀ heterohydrocarbyl group and p and q, independent of each other may be 0 or an integer of from 1 to 3.

9. Energy storage device comprising at least one electrode in accordance with claims 1 to 8.

10. Energy storage device in accordance with claim 9 which is a rechargeable or non-rechargeable battery, a redox flow battery a supercapacitor or a hybrid supercapacitor.

11. Energy storage device in accordance with claim 10 which is a recheargeable battery selected from the group consisting of lithium-ion batteries, lithium-polymer batteries, sodium ion batteries, magnesium ion batteries and calcium ion batteries.

12. Use of modified carbon materials comprising organic redox moieties covalently attached to the surface for the manufacture of electrodes for energy storage devices, in particular rechargeable and non-rechargeable batteries and supercapacitors.

13. Use of modified carbon materials in accordance with claim 7 wherein the modified carbon material is used in combination with inorganic materials, redox polymers or a combination of inorganic materials and redox polymers.

14. Use of modified carbon materials comprising organic redox moieties covalently attached to the surface in the catholyte of redox-flow batteries.

15. Azide compounds represented by the general formula (3) 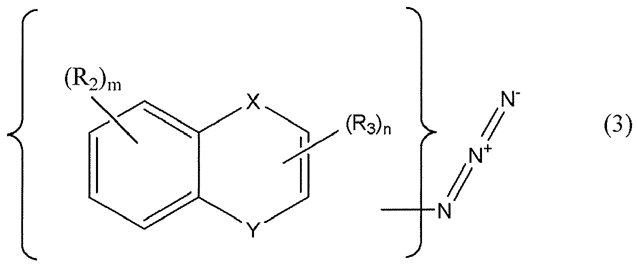
wherein the azide group may be attached at any carbon atom and wherein X and Y, which may be the same or different, represent NR₁, O, S, SO or SO₂, and R₁ to R₃, which may be the same or different at each occurrence represent a C₁-C₃₀ hydrocarbyl group or a C₁-C₃₀ heterohydrocarbyl group, m may be 0 or an integer of from 1 to 3 and n may be 0, 1 or 2 or.

16. Azide compounds represented by the general formula (4) 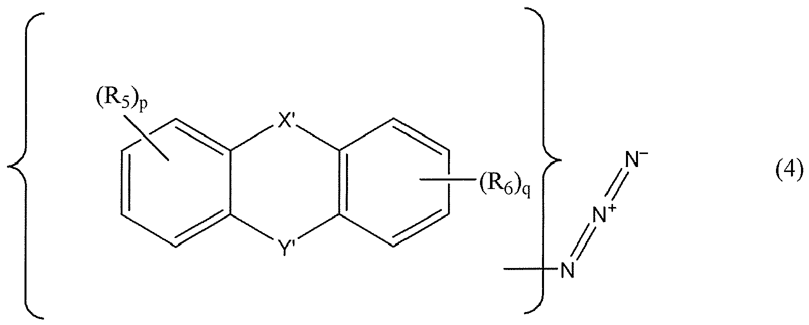
wherein the azide group may be attached to any of the carbon atoms in any of the aromatic rings and wherein X, X', Y and Y', which may be the same or different, represent NR₁, NR₄, O, S, SO or SO₂, and R₁ to R₆, which may be the same or different at each occurrence represent a C₁-C₃₀ hydrocarbyl group or a C₁-C₃₀ heterohydrocarbyl group and n, m, p and q, independent of each other may be 0 or an integer of from 1 to 3.
